# EUROPEAN PATENT SPECIFICATION

(11) **EP 1 596 891 B1**
(45) Date of publication and mention of the grant of the patent: **23.01.2008**
(21) Application number: 03715345.9
(22) Date of filing: 12.03.2003
(51) Int. Cl.: A61L 9/03, A61L 9/02, B60H 3/00

(54) **ELECTRIC DEVICE FOR SCENTING THE INTERIOR OF VEHICLES**
ELEKTRISCHES GERÄT ZUM BEDUFTEN DES INNENRAUMES VON FAHRZEUGEN
DISPOSITIF ELECTRIQUE PERMETTANT DE PARFUMER L'INTERIEUR DE VEHICULES

(30) Priority: 15.03.2002 IT PI20020015
(43) Date of publication of application: 23.11.2005
(73) Proprietor: Eureka S.N.C., 52100 Arezzo (IT); SARONG S.p.A., 42046 Reggiolo (IT)
(72) Inventor: FANTONI, Adriano, I-52100 Arezzo (IT); MAGRINI, Giancarlo, I-52100 Arezzo (IT); BORRI, Gianmatteo, I-52100 Arezzo (IT)
(74) Representative: Turini, Laura
(86) International application number: PCT/IT2003/000152
(87) International publication number: WO 2003/077961

(56) References cited:
- GB-A- 2 352 973
- US-A- 4 544 592
- US-A- 5 373 581
- US-A- 5 788 931
- US-A- 6 089 947
- US-B1- 6 197 263
- US-B1- 6 289 176
- DATABASE WPI Section Ch, Week 198838 Derwent Publications Ltd., London, GB; Class A23, AN 1988-266465 XP002246852 & JP 63 097168 A (NIPPON OIL SEAL IND CO LTD), 27 April 1988 (1988-04-27)

## Description

### Technical Field

The present invention relates to the technical sector of the production of accessories for cars and particularly of devices used to scent the interior of a car or other vehicles.

### Background Art

Several car scenting devices are nowadays used, principally constituted by items of different shape, made of a material filled with perfume, so that, once partially or totally removed from their protective film, they diffuse perfume in the air. Such a kind of device has however the drawback that it doesn't regulate the appropriate gradation of perfume and it keeps on emitting perfume in any moment, when the vehicle is on as well as off, and the user cannot do anything to stop this process.

Other scenting devices also exist, consisting of a liquid perfume receptacle holding a sponge with its upper part placed in front of an accessory that is applied on the heaters, so that when the air comes into the vehicle, it makes the perfume diffused. But also this product has relevant drawbacks: first of all, the high cost of the refill and its wear by evaporation, in any case, without considering that accidental falls might spill the liquid and imply relative negative consequences.

Furthermore, from US-A-6289176, a device for scenting the interior of vehicles is known.

### Disclosure of invention

The present invention principally aims at supplying a device able to diffuse a perfect perfume in the interior, adjustable according to the user's need, having the characteristics described in the separate claims. Other characteristics of this invention are the object of dependent claims.

The advantages resulting from the present invention essentially consist of the fact that it's possible to make the interior of a vehicle scented in any moment, but only if desired; that the device is clean and small, easy to use and difficult to wear; that the cost of the refills of perfume is reduced; that its working system allows to make the interior scented, using only a small amount of perfume; that it's powered by the 12V/24V circuit of the same vehicle.

These and further advantages of the present invention can be better understood by every expert in this field by reading the following description and referring to the enclosed drawings.

Reduced to its essential structure and with reference to the figures of the enclosed drawings, a device for scenting the interior of vehicles, according to the invention, comprises:
- means to scent the interior of a vehicle, with a perfumed capsule sending out vapours only after its heating at a prefixed temperature;
- means to heat said capsule by an electric or electronic component able to produce heat, connected to a circuit powered by the circuit of the vehicle it is connected to;
- means to turn on and off the device, with a switch installed in the circuit, possibly connected to a luminous led informing the user about the on/off status of the same device.

The capsule is inserted into the device from the outside by a suitable opening (6) and is placed on a support (16) inside the device near the heating component, so that when said component heats, the warmth rapidly transfers to the capsule too.

The scented capsule is made of plastic material holding perfumed gel and its upper surface is made of SILICONE material with cells permitting the perfume to exude.

Conveniently, in a practical example, the component able to produce heat consists of a resistance (14) connected to the electric circuit.

Conveniently, the device can be either integrated into the vehicle or separated as an accessory to be connected to the car electric circuit when used.

Conveniently, in one part (1) the device takes a pin shape equipped with a rounded tip (2), a prod (7) and two flaps (3), so that it's fitted into the socket of the cigarette lighter in order to take current.

Conveniently, this device works with a 12V/24V current.

The device also includes a safety fuse (8) that is applied to the circuit in order to protect it from possible overcharges. Said fuse can be removed and replaced in case it's broken.

Conveniently, this device has a series of holes on one or more parts of its surface, permitting the perfume to come out better.

This is the way this device works:
Fitting the device into the socket of the car cigarette lighter, it turns on, sets the led on, and starts the heat of the resistance or other electric/electronic component able to produce heat, provided that the switch is on. Once the selected temperature has been reached, the process of diffusing the perfume held into the scented capsule starts and ends only when the device is turned off by its switch or by its removing from the socket of the cigarette lighter.
In practice, the manufacturing details may, however, vary as regards shape, size, position of elements, and type of materials used, but still remain within the range of the idea proposed as a solution and, consequently, within the limits of the protection granted by this patent for invention.

### Brief description of drawings

The enclosed drawings are given as practical examples of the invention, but are not to be considered restrictive.

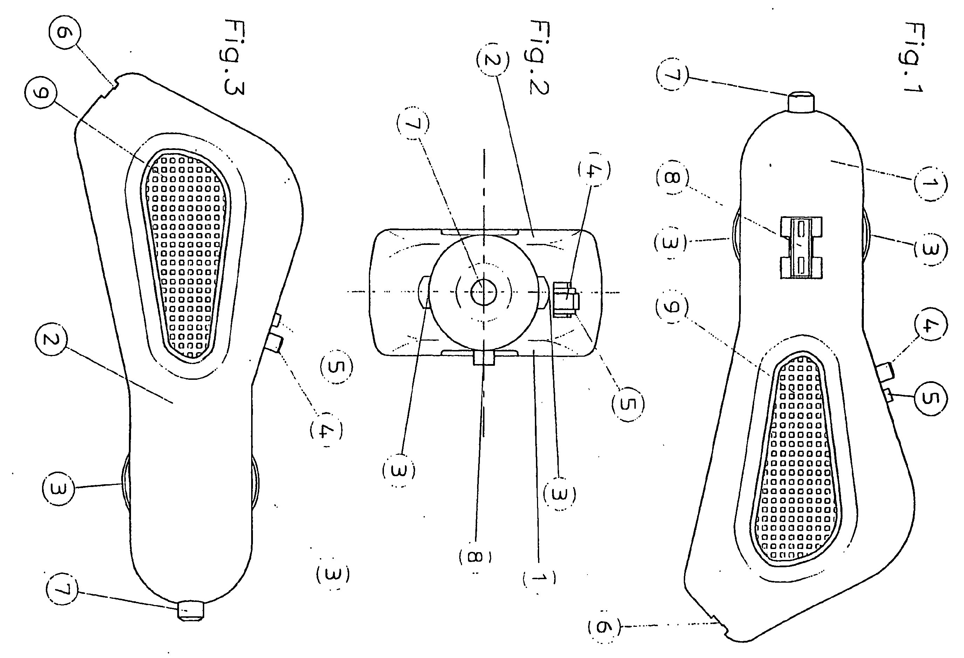

- Fig. 1 shows an external view of the device with the plastic body having an end part (1) to be fitted into the socket of the car cigarette lighter, so that by means of its tip (7) and its flaps (3) it powers the internal circuit. The same figure also shows the led (5), the switch (4) used to turn the device on/off, the opening (6) inside which the scented capsule is inserted and a surface with holes (9) where perfume exudes.
- Fig. 2 shows the front view of the same device as Fig. 1.
- Fig. 3 shows the opposite side of the same device as Fig.1.

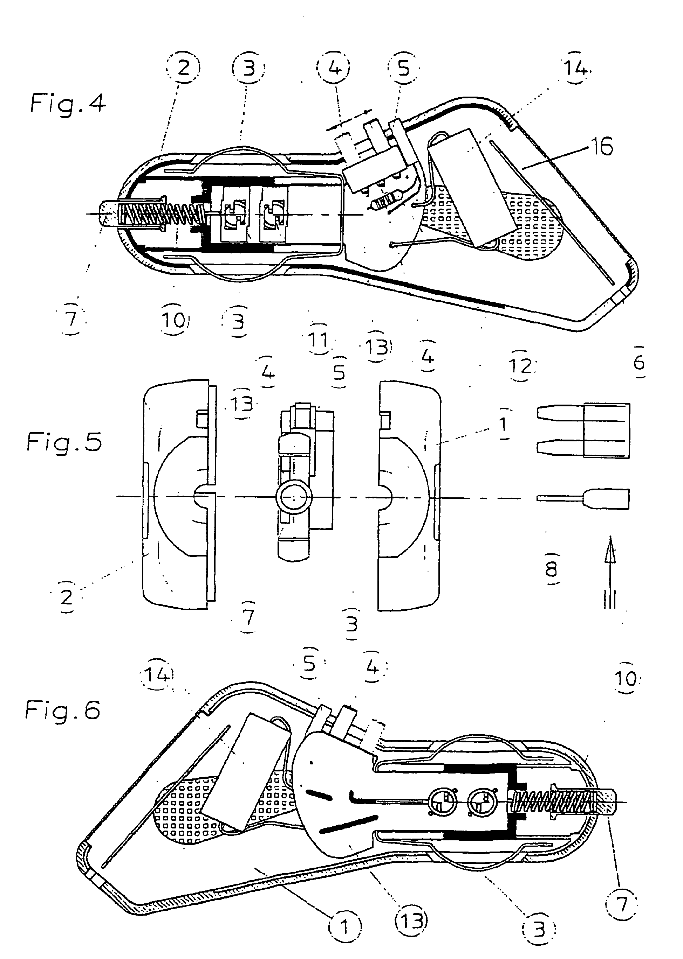

- Fig. 4 shows the inside of the device, with an end rounded part (2) to be fitted into the socket of the car cigarette lighter, the flaps (3) and the tip (7) joined to a spring (10) connected to the circuit comprising two holders (11) inside which the wires of the fuse (8) enter. The circuit is connected to a switch (4) joined to a led (5) and to a power resistance (12). The circuit is also connected to a resistance (14) having the function to heat the perfume placed over the support (16) fitting it through the opening (6).
- Fig. 5 shows the front exploded view of the device.
- Fig. 6 shows the opposite inside of the device.

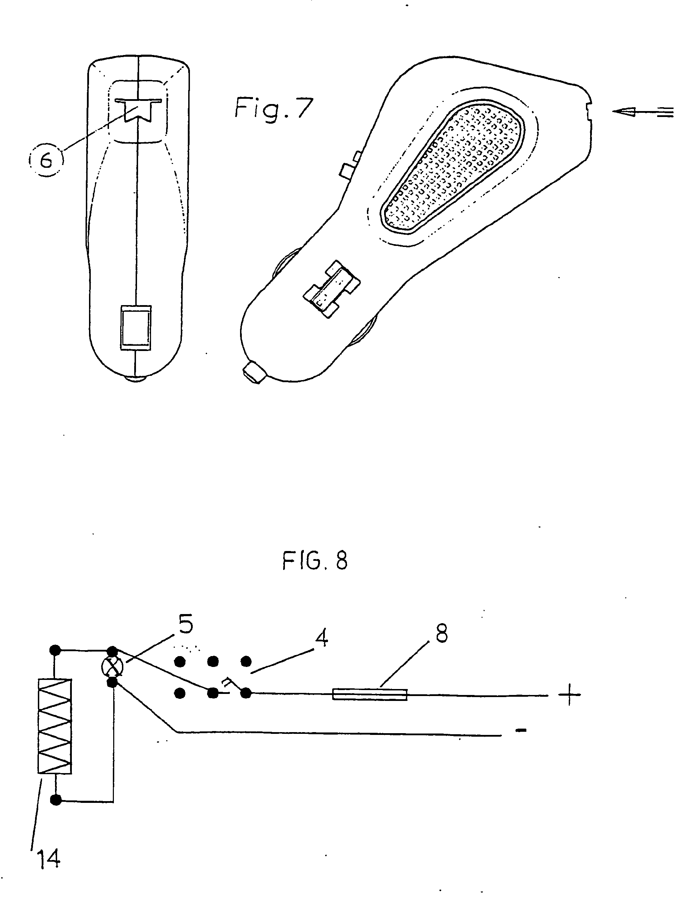

- Fig. 7 shows the final device, highlighting the opening (6) through which the scented capsule is inserted.
- Fig. 8 shows the electric circuit of the device, with the resistance (14), the switch (4), the led (5) and the protective fuse (8).

## Claims

1. Device for scenting the interior of vehicles by heating a substance capable of sending out vapours, comprising :
- a capsule made of plastic material holding perfumed gel, having an upper surface made of SILICONE material with cells permitting the perfume to exude, which is inserted into the device from the outside by a suitable opening (6) and is placed on a support (16) inside the device near an electric or electronic component able to produce heat, the electric or electronic component being connected to a circuit powered by the circuit of the vehicle
- means to turn on and off the device, with a switch installed in the circuit, preferably connected to a luminous led informing the user about the on/off status of the same devices,
- a safety fuse (8), which can be removed and replaced, applied to the circuit in order to protect it from possible overcharges.

2. Device according to claim 1, **characterized in that** it is made of a plastic body with an end rounded part (2) to be fitted into the socket of A car cigarette lighter, flaps 13) and A tip (7) of the end rounded part (2) are joined to a spring (10) connected to the circuit comprising two holders (11) inside which the wires of the fuse (8) enter, the circuit is connected to a switch (4) joined to SAID led (5) and to a power resistance (12) and additionally connected to a resistance (14) having the function to heat the perfume which was placed over the support (16) by fitting it through the opening (6).

3. Device according to claim 1, **characterized in that** it works with a 12V/24V current.

4. Device according to claim 1, **characterized in that** it has a series of holes on one or more parts of its surface, permitting the perfume to come out better.

## Patentansprüche

1. Vorrichtung zum Beduften des Inneren von Fahrzeugen, indem eine Substanz erhitzt wird, die in der Lage ist, Dämpfe auszusenden, mit:
- einer Kapsel, die aus einem Kunststoffmaterial hergestellt ist, das ein parfümiertes Gel enthält, mit einer oberen Fläche, die aus einem Silikon-Material gemacht ist, wobei Zellen das Parfüm ausströmen können, welche Kapsel in die Vorrichtung von außen über eine geeignete Öffnung (6) eingebracht und auf einen Träger (16) platziert ist, der innerhalb der Vorrichtung nahe einer elektrischen oder elektronischen Komponente ist, um Wärme zu produzieren, wobei die elektrische oder elektronische Komponente mit einer Schaltung verbunden ist, die von der Schaltung des Fahrzeugs mit Energie versorgt wird;
- einem Mittel zum Ein- und Ausschalten der Vorrichtung, wobei ein Schalter in der Schaltung vorgesehen ist, vorzugsweise mit einer leuchtenden LED verbunden ist, die den Benutzer über den Ein/Aus-Status der gleichen Vorrichtung informiert;
- einer Sicherheitssicherung (8), die entfernt und ersetzt werden kann und mit der Schaltung verbunden ist, um sie gegenüber möglichen Überspannungen zu schützen.

2. Vorrichtung nach Anspruch 1, **dadurch gekennzeichnet, dass** sie aus einem Kunststoffkörper hergestellt ist, der ein endabgerundetes Teil (2) aufweist, das in den Sockel eines Fahrzeugzigarettenanzünders passt, Klappen (3) und eine Spitze (7) des endabgerundeten Teils (2) mit einer Feder (10) verbunden sind, die mit der Schaltung verbunden ist und zwei Halter (11) aufweist, in die Drähte der Sicherung (8) hineingehen, wobei die Schaltung mit einem Schalter (4) verbunden ist, der mit der LED (5) verknüpft ist, und mit einem Leistungswiderstand (12) und zusätzlich mit einem Widerstand (14) verbunden ist, der die Funktion hat, das Parfüm zu erhitzen, das über dem Träger (16) platziert wurde, indem es durch die Öffnung (6) eingepasst wurde.

3. Vorrichtung nach Anspruch 1, **dadurch gekennzeichnet, dass** sie mit einer 12 V/24 V-Spannung arbeitet.

4. Vorrichtung nach Anspruch 1, **dadurch gekennzeichnet, dass** sie eine Reihe von Löchern in einen oder mehreren Teilen ihrer Oberfläche aufweist, die ein besseres Ausströmen des Parfüms ermöglichen.

## Revendications

1. Dispositif pour parfumer l'intérieur de véhicules par chauffage d'une substance apte à émettre des vapeurs, comprenant :
- une capsule faite de matière plastique, contenant un gel parfumé, ayant une surface supérieure faite d'un matériau silicone avec des alvéoles permettant au parfum d'exsuder, qui est insérée dans le dispositif depuis l'extérieur par une ouverture adaptée (6) et qui est placée sur un support (16) à l'intérieur du dispositif, à proximité d'un composant électrique ou électronique apte à produire de la chaleur, le composant électrique ou électronique étant raccordé à un circuit alimenté par le circuit du véhicule,
- des moyens pour mettre en marche et arrêter le dispositif, avec un commutateur installé dans le circuit, de manière préférée raccordé à une DEL lumineuse informant l'utilisateur du statut marche/arrêt dudit dispositif,
- un fusible de sécurité (8), qui peut être retiré et remplacé, appliqué au circuit de manière à le protéger d'éventuelles surcharges.

2. Dispositif selon la revendication 1, ***caractérisé en ce qu'il*** est fait d'un corps en matière plastique avec une partie terminale arrondie (2) à insérer dans la douille d'un allume-cigare du véhicule, des ailerons (3) et une pointe (7) de la partie terminale arrondie (2) sont associés à un ressort (10) raccordé au circuit comprenant deux supports (11) à l'intérieur desquels entrent les fils du fusible (8), le circuit est relié à un commutateur (4) associé à ladite DEL (5) et à une résistance électrique (12), et en plus relié à une résistance (14) ayant pour fonction de chauffer le parfum qui a été placé sur le support (16) en l'insérant par l'ouverture (6).

3. Dispositif selon la revendication 1, ***caractérisé en ce qu'il*** fonctionne avec un courant 12V/24V.

4. Dispositif selon la revendication 1, ***caractérisé en ce qu'il*** comporte une série de trous ménagés sur une ou plusieurs parties de sa surface, permettant au parfum de mieux s'échapper.
